(19) Europäisches Patentamt
European Patent Office
Office européen des brevets

(11) Veröffentlichungsnummer: **0 018 585**
**B1**

(12) **EUROPÄISCHE PATENTSCHRIFT**

(45) Veröffentlichungstag der Patentschrift: **08.02.84**

(21) Anmeldenummer: **80102181.7**

(22) Anmeldetag: **23.04.80**

(51) Int. Cl.³: **C 07 D 233/96, A 01 N 43/50**

# (54) Parabansäurederivate, Herbizide, die diese Verbindungen enthalten, und Verfahren zur Herstellung der Herbizide.

(30) Priorität: **25.04.79 DE 2916647**

(43) Veröffentlichungstag der Anmeldung: **12.11.80 Patentblatt 80/23**

(45) Bekanntmachung des Hinweises auf die Patenterteilung: **08.02.84 Patentblatt 84/6**

(84) Benannte Vertragsstaaten: **AT BE CH DE FR GB IT LI LU NL SE**

(56) Entgegenhaltungen:
DE - A - 1 670 943
US - A - 2 895 817
US - A - 3 418 334

**Chemical abstracts, Band 87, Nr. 12, 19. September 1977, Zusammenfassung Nr. 92583q, Seite 524, Columbus, Ohio, US, G.S. SUPIN et al.: "Polarography of N'N-disubstituted derivatives of parabanic acid"**

**Die Akte enthält technische Angaben, die nach dem Eingang der Anmeldung eingereicht wurden und die nicht in dieser Patentschrift enthalten sind.**

(73) Patentinhaber: **BASF Aktiengesellschaft
Carl-Bosch-Strasse 38
D-6700 Ludwigshafen (DE)**

(72) Erfinder: **Schirmer, Ulrich, Dr.
Berghalde 79
D-6900 Heidelberg 1 (DE)**
Erfinder: **Becker, Rainer, Dr.
Sonnenwendstrasse 83a
D-6702 Bad Duerkheim 1 (DE)**
Erfinder: **Wuerzer, Bruno, Dr., Dipl.-Landwirt
Ruedigerstrasse 13
D-6701 Otterstadt (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 018 585 B1

## Parabansäurederivate, Herbizide, die diese Verbindungen enthalten, und Verfahren zur Herstellung der Herbizide

Die vorliegende Erfindung betrifft neue wertvolle Parabansäurederivate mit herbizider Wirkung, Herbizide, die diese Verbindungen enthalten, Verfahren zur Bekämpfung unerwünschten Pflanzenwuchses mit diesen Verbindungen und Verfahren zur Herstellung der Herbizide.

Unsubstituierte 3-Phenyl- und durch Halogen substituierte 3-Phenylparabansäuren sind als Herbizide bekannt (US—A—2 895 817). Dort wird ihre Verwendung zur totalen Beseitigung von unerwünschter Vegetation beschrieben. Besonders hervorgehoben wird die Bekämpfung von "Crabgrass" (Digitaria spp., Bluthirse) in Rasen durch 1-Methyl-3-phenylparabansäure und 1-Methyl-3-(p-chlorphenyl)parabansäure. Auch die Möglichkeit, Samenunkräuter in Mais zu bekämpfen wird demonstriert. 1,3-Bis-(p-phenoxyphenyl)parabansäure mit herbiziden Eigenshaften ist aus DE—A—1670943 bekannt.

Es wurde nungefunden, daß neue Parabansäurederivate der allgemeinen Formel

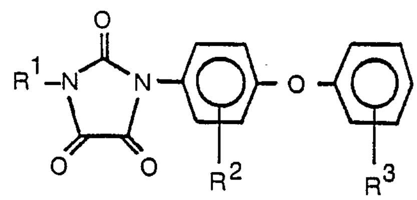

in der

$R^1$ Alkyl mit 1 bis 4 C-Atomen,

$R^2$ Halogen und

$R^3$ Alkyl mit 1 bis 3 C-Atomen, Halogen, Methoxy, Trifluoromethoxy, Difluormethoxy oder Chlortrifluorethoxy bedeutet, einerseits eine so breite

und starke herbizide Wirkung besitzen, daß man sie zur totalen Bekämpfung unerwünschter Pflanzen oder in mehrjährigen Dauerkulturen einsetzen kann, andererseits aber eine überraschende Verträglichkeit für verschiedene im einjährigen Anbau angesäte Kulturpflanzen haben.

Der in der allgemeinen Formel angeführte Rest $R^3$ hat folgende Bedeutungen:

Alkyl mit 1 bis 3 C-Atomen (z.B. Methyl, Isopropyl), Halogen (z.B. Fluor, Chlor, Brom oder Jod), Methoxy, Trifluormethoxy, Difluormethoxy, Chlortrifluorethoxy.

Die neuen Verbindungen können beispielsweise nach folgenden Verfahren hergestellt werden, wobei die Reste $R^1$ und R die oben angegebene Bedeutung haben.

I

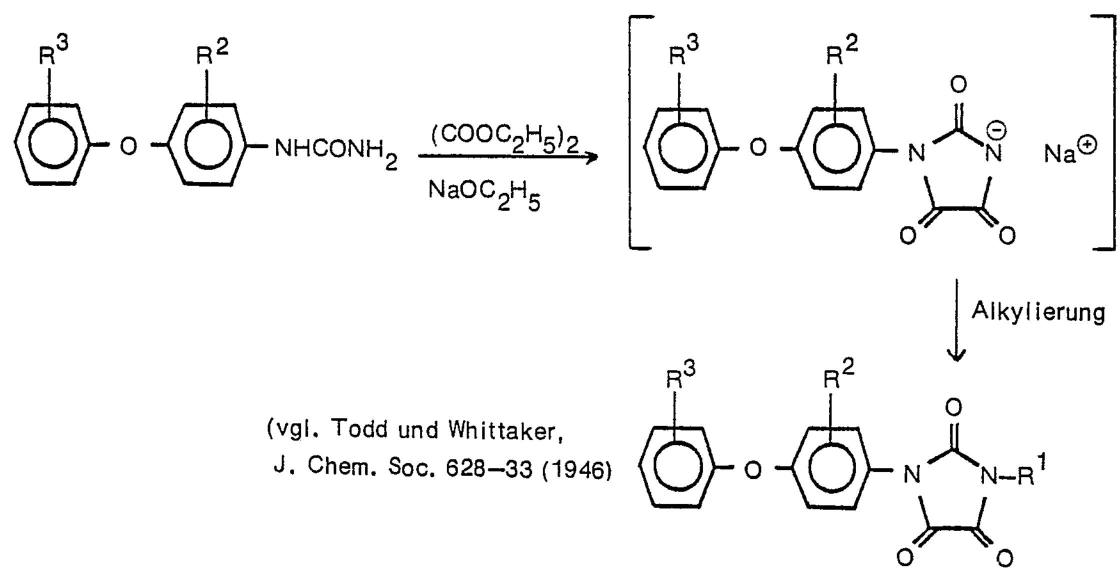

II 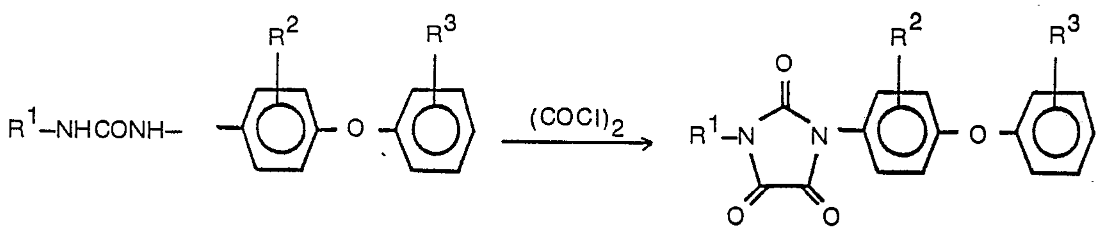


(vgl. US—A—3 418 334

Nachfolgend wird der bevorzugte Syntheseschritt genauer beschrieben:

1-Aryl-3-aliphatenharnstoffe (erhältlich durch Umsetzung von aliphatischen Isocyanaten mit aromatischen Aminen bzw. durch Umsetzung von Arylisocyanaten mit aliphatischen Aminen (z.B. DE—A—2 048 660, DE—A—2 558 078) werden in einem unter den Reaktionsbedingungen inerten Lösungsmittel, z.B. Kohlenwasserstoffe (Ligroin, Benzin, Toluol, Cyclohexan), Halogenkohlenwasserstoffe (Methylenchlorid, Chloroform, Chlorbenzol, Brombenzol) oder Nitrokohlenwasserstoff (Nitrobenzol) mit äquimolaren oder überschüssigen Mengen an Oxalylchlorid zwischen 30 und 180°C umgesetzt bis keine Chlorwasserstoffentwicklung mehr auftritt.

In dieser Weise können beispielsweise folgende Verbindungen hergestellt werden.

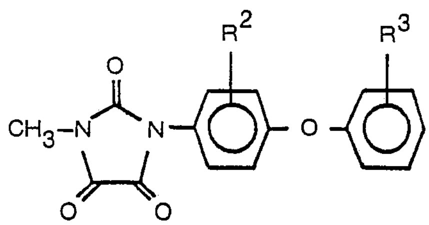

| Nr. | $R^2$ | $R^3$ | Fp °C |
|---|---|---|---|
| 1 | 3-Cl | 4′-methyl | 179—182 |
| 2 | 3-Cl | 3′-methoxy | 150—151 |
| 3 | 3-F | 4′-methoxy | 195—201 |
| 4 | 3-Cl | 4′-methoxy | 150—152 |
| 5 | 3-Cl | 4′-chlor | 184—187 |

Es werden ferner beispielsweise die folgenden Wirkstoffe genannt:

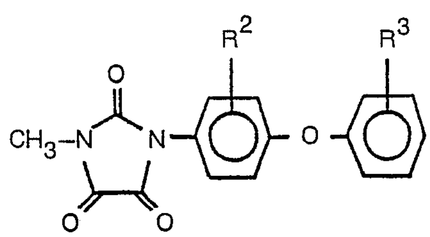

| Nr. | $R^2$ | $R^3$ |
|---|---|---|
| 6 | 2-F | 4′-fluor |
| 7 | 2-F | 3′-methoxy |
| 8 | 3-F | 4′-fluor |
| 9 | 3-Cl | 4′-difluormethoxy |

Der Einfluß der erfindungsgemäßen Verbindungen auf das Wachstum von unerwünschten Pflanzen und Kulturen wird in nachfolgenden Gewächshausversuchen demonstriert.

Als Kulturgefäße dienten Plastikblumentöpfe mit 300 $cm^3$ Inhalt und lehmiger Sand mit etwa 1,5% Humus als Substrat. Die Samen der Testpflanzen entsprechend Tabelle 1 wurden nach Arten getrennt flach eingesät. Unmittelbar danach erfolgte bei Vorauflaufanwendung das Aufbringen der Wirkstoffe auf die Erdoberfläche. Sie wurden hierbei in Wasser als Verteilungsmittel suspendiert oder emulgiert und mittels fein verteilender Düsen gespritzt. Nach dem Aufbringen der Mittel wurden die Gefäße leicht beregnet, um Keimung und Wachstum in Gang zu bringen und auch gleichzeitig die chemischen Mittel zu aktivieren. Danach deckte man die Gefäße mit durchsichtigen Plastikhauben ab, bis die Pflanzen angewachsen waren. Diese Abdeckung bewirkte ein gleichmäßiges Keimen der Testpflanzen, sofern dies nicht durch die Chemikalien beeinträchtigt wurde.

Zum Zwecke der Nachauflaufbehandlung zog man die Pflanzen je nach Wuchsform in den Versuchsgefäßen erst bis zu einer Höhe von 3 bis 10 cm an und behandelte sie dann. Eine Abdeckung unterblieb. Die Aufstellung der Versuche erfolgte im Gewächshaus, wobei für wärmeliebende Arten heißere Bereiche von 25 bis 40°C und für solche gemäßigter Klimate 15 bis 30°C bevorzugt wurden. Die Versuchsperiode erstreckte sich über 3 bis 6 Wochen. Während dieser Zeit wurden die Pflanzen gepflegt und ihre Reaktion auf die einzelnen Behandlungen ausgewertet. Die folgenden Tabellen enthalten die Prüfsubstanzen, die jeweiligen Dosierungen in kg/ha Aktivsubstanz und die Testpflanzen.

Bewertet wird nach einer Skala von 0 bis 100. Dabei bedeutet 0 keine Schädigung oder normalen Auflauf und 100 kein Aufgang der Pflanzen bzw. völlige Zerstörung zumindest der oberirdischen Sproßteile.

Gegenüber den neuen herbiziden Verbindungen zeigen landwirtschaftliche Kulturpflanzen eine überraschend gute Verträglichkeit. Die Applikation kann im Vorauflaufverfahren oder im Nachauflaufverfahren erfolgen. Dabei können die Mittel auf den Standort aufgebracht werden bevor die unerwünschten Pflanzen aus den Samen gekeimt oder aus vegetativen Pflanzenteilen ausgetrieben haben oder sie werden auf die Blätter der unerwünschten Pflanzen und der Kulturpflanzenverträglichkeit appliziert. Eine weitere Ausbringungstechnik besteht darin, daß die Wirkstoffe mit Hilfe der Spritzgeräte so gespritzt werden, daß die Blätter empfindlicher Kulturpflanzen nach Möglichkeit nicht getroffen werden, während die Wirkstoffe auf die darunterliegende Bodenfläche oder dort wachsende unerwünschte Pflanzen gelangen (post-directed, layby). Die Aufwandmengen betragen je nach Jahreszeit und Wachstumsstadium 0,1 bis 15 kg/ha und mehr, wobei sich die höheren Dosen besonders zur totalen Bekämpfung von Vegetationen eignen

In Anbetracht der Vielseitigkeit der Applikationsmethoden können die erfindungsgemäßen Mittel oder diese enthaltenden Mischungen außer bei den in den Tabellen aufgeführten Nutzpflanzen noch in einer weiteren großen Zahl von Kulturen zur Beseitigung unerwünschten Pflanzenwuchses eingesetzt werden.

Im einzelnen seien folgende Nutzpflanzen genannt:

| Botanischer Name | Deutscher Name | Englischer Name |
|---|---|---|
| Allium cepa | Küchenzwiebel | onions |
| Ananas comosus | Ananas | pineapple |
| Arachis hypogaea | Erdnuß | peanuts (groundnuts) |
| Asparagus officinalis | Spargel | asparagus |
| Avena sativa | Hafer | oats |
| Beta vulgaris spp. altissima | Zuckerrübe | sugarbeets |
| Beta vulgaris spp. rapa | Futterrübe | fooder beets |
| Beta vulgaris spp. esculenta | Rote Rübe | table beets, red beets |
| Brassica napus var. napus | Raps | rape seed |
| Brassica napus var. napobrassica | Kohlrübe | |
| Brassica napus var. rapa | Weiße Rübe | turnips |
| Brassica rapa var. silvestris | Rübsen | |

| Botanischer Name | Deutscher Name | Englischer Name |
|---|---|---|
| Camellia sinensis | Teestrauch | tea plants |
| Carthamus tinctorius | Saflor-Färbedistel | safflower |
| Carya illinoinensis | Pekannußbaum | pecan trees |
| Citrus limon | Zitrone | lemon |
| Citrus maxima | Pampelmuse | grapefruits |
| Citrus reticulata | Mandarine | |
| Citrus sinensis | Alfelsine, Orange | orange trees |
| Coffea arabica (Coffea canephora, Coffea liberica) | Kaffee | coffee plants |
| Cucumis melo | Melone | melons |
| Cucumis sativus | Gurke | cucumber |
| Cynodon dactylon | Bermudagras | Bermudagrass in turfs and lawns |
| Daucus carota | Möhre | carrots |
| Elaeis guineensis | Ölpalme | oil palms |
| Fragaria vesca | Erdbeere | strawberries |
| Glycine max | Sojabohne | soybeans |
| Gossypium hirsutum (Gossypium arboreum Gossypium herbaceum Gossypium vitifolium) | Baumwolle | cotton |
| Helianthus annuus | Sonnenblume | sunflowers |
| Helianthus tuberosus | Topinambur | |
| Hevea brasiliensis | Parakautschukbaum | rubber plants |
| Hordeum vulgare | Gerste | barley |
| Humulus lupulus | Hopfen | hop |
| Ipomoea batatas | Süßkartoffeln | sweet potato |
| Juglans regia | Walnußbaum | walnut trees |
| Lactuca sativa | Kopfsalat | lettuce |
| Lens culinaris | Linse | lentils |
| Linum usitatissimum | Faserlein | flax |
| Lycopersiocan lycopersicum | Tomate | tomato |
| Malus spp. | Apfel | apple trees |
| Manihot esculenta | Maniok | cassava |

| Botanischer Name | Deutscher Name | Englischer Name |
|---|---|---|
| Medicago sativa | Luzerne | alfalfa (lucerne) |
| Mentha piperita | Pfefferminze | peppermint |
| Musa spp. | Obst- u. Mehlbanane | banana plants |
| Nicotiana tabacum (N. rustica) | Tabak | tobacco |
| Olea europaea | Ölbaum | olive trees |
| Oryza sativa | Reis | rice |
| Panicum miliaceum | Rispenhirse | |
| Phaseolus lunatus | Mondbohne | limabeans |
| Phaseolus mungo | Erdbohne | mungbeans |
| Phaseolus vulgaris | Buschbohnen | snapbeans, green beans, dry beans |
| Pennisetum glaucum | Perl- oder Rohrkolbenhirse | |
| Petroselinim crispum spp. tuberosum | Wurzelpetersilie | parsley |
| Picea abies | Rotfichte | Norway spruce |
| Abies alba | Weißtanne | fire |
| Pinus spp. | Kiefer | pine trees |
| Pisum sativum | Gartenerbse | English peas |
| Prunus avium | Süßkirsche | cherry trees |
| Prunus domestica | Pflaume | plum trees |
| Prunus dulcis | Mandelbaum | almond trees |
| Prunus persica | Pfirsich | peach trees |
| Pyrus communis | Birne | pear trees |
| Ribes sylvestre | Rote Johannisbeere | red currants |
| Ribes uva-crispa | Stachelbeere | |
| Ricinus communis | Rizinus | |
| Saccharum officinarum | Zuckerrohr | sugar cane |
| Secale cereale | Roggen | rye |
| Sesamum indicum | Sesam | Sesame |
| Solanum tuberosum | Kartoffel | Irish potato |
| Sorghum bicolor (s. vulgare) | Mohrenhirse | sorghum |
| Sorghum dochna | Zuckerhirse | |
| Spinacia oleracea | Spinat | spinach |

| Botanischer Name | Deutscher Name | Englischer Name |
|---|---|---|
| Theobroma cacao | Kakaobaum | cacao plants |
| Trifolium pratense | Rotklee | red clover |
| Triticum aestivum | Weizen | wheat |
| Vaccinium carymbosum | Kulturheidelbeere | blueberry |
| Vaccinium vitis-idaea | Preißelbeere | cranberry |
| Vicia faba | Pferdebohnen | tick beans |
| Vigna sinensis (V. unguiculata) | Kuhbohne | cow peas |
| Vitis vinifera | Weinrebe | grapes |
| Zea mays | Mais | Indian corn, sweet corn, maize |

Die Parabansäurederivate können unter sich und mit zahlreichen Vertretern anderer herbizider oder wachstumsregulierender Wirkstoffgruppen gemischt und gemeinsam ausgebracht werden. Beispielsweise kommen als Mischungskomponenten Diazine, N-Phenyl-carbamate, Thiolcarbamate, Diurethane, Halogencarbonsäuren, Phenoxyfettsäuren, Triazine, Amide, Harnstoffe, Diphenyläther, Triazone, Uracile, Benzofuranderivate und andere in Betracht. Solche Kombinationen dienen zur Verbreiterung des Wirkungsspektrums und erzielen zuweilen synergistische Effekte.

Außerdem ist es nützlich, die neuen erfindungsgemäßen Verbindungen allein oder in Kombinationen mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse sind ferner die Mischbarkeit mit Mineralstofflösungen, welche zur Behebung von Ernährungs- oder Spurenelementmängeln eingesetzt werden. Zu den Einzelwirkstoffen oder Mischungen können auch Öle verschiedenen Typs, Netz- oder Haftmittel sowie Antischaummittel zugesetzt werden.

TABELLE 1 (fortsetzung)

Liste der Testpflanzen

| Botanischer Name | Deutsche Bezeichnung | Englische Bezeichnung |
|---|---|---|
| Abutilon theophrasti | Chinesischer Hanf | velvet leaf |
| Amaranthus retroflexus | Zurückgekrümmter Fuchsschwanz | redroot pigweed |
| Arachis hypogaea | Erdnuß | peanuts (groundnuts) |
| Avena sativa | Hafer | oats |
| Beta vulgaris | Zuckerrübe | sugarbeets |
| Centaurea cyanus | Kornblume | cornflower |
| Chrysanthemum segetum | Saatwucherblume | corn marigold |
| Cyperus esculentus | Erdmandel | yellow nutsedge |
| Echinochloa crus galli | Hühnerhirse | barnyardgrass |
| Euphorbia geniculata | Südamerikanische Wolfsmilchart | Southamerican member of spurge family |
| Galium aparine | Klettenlabkraut | catchweed bedstraw |
| Gossypium hirsutum | Baumwolle | cotton |
| Ipomoea spp. | Prunkwindearten | morningglory |
| Lolium multiflorum | Ital. Raygras | annual ryegrass |
| Matricaria spp. | Kamillearten | chamomile |
| Mentha piperita | Pfefferminze | Peppermint |
| Nicandra physaloides | Giftbeere | apple-o-Peru |
| Sesbania exaltata | Turibaum | hemp sesbania (coffeeweed) |
| Sinapis alba | Weißer Senf | white mustard |
| Solanum nigrum | schwarzer Nachschatten | black nightshade |
| Sorghum bicolor | Mohrenhirse (Kulturhirse) | sorghum |
| Stellaria media | Vogelsternmiere | chickweed |
| Triticum aestivum | Weizen | wheat |
| Veronica perisca | Persischer Ehrenpreis | birdseye speedwell |

Die Anwendung erfolgt z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten und Öldispersionen

kommen Mineralölfraktionen von mittlerem bis hohem Siedepunkt, wie Kerosin oder Dieselöl, ferner Kohlenteeröle usw., sowie Öle pflanzlichen und tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate, zum Beispiel Methanol, Äthanol, Propanol, Butanol, Chloroform, Tetrachlorkohlenstoff, Cyclohexanol, Cyclohexanon, Chlorbenzol, Isophoron usw., stark polare Lösungsmittel, wie z.B. Dimethylformamid, Dimethylsulfoxid, N-Methylpyrrolidon, Wasser in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

An oberlächenaktiven Stoffen sind zu nennen: Alkali-, Erdalkali-, Ammoniumsalze von Ligninsulfonsäure, Naphthalinsulfonsäuren, Phenolsulfonsäuren, Alkylarylsulfonate, Alkylsulfate, Alkylsulfonate, Alkali- und Erdalkalisalze der Dibutylnaphthalinsulfonsäure, Lauryläthersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykoläther, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyäthylenoctylphenoläther, äthoxyliertes Isooctylphenol-, Octylphenol-, Nonylphenol, Alkylphenolpolyglykoläther, Tributylphenylpolyglykoläther, Alkylarylpolyätheralkohole, Isotridecylalkohol, Fettalkoholäthylenoxid-Kondensate, äthoxyliertes Rizinusöl, Polyoxyäthylenalkyläther, äthoxyliertes Polyoxypropylen, Laurylalkoholglykolätheracetal, Sorbitester, Lignin, Sulfitablaugen und Methylcellulose.

Pulver, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden wie Kieselsäuren, Silikate, Talkum, Kaolin, Kalkstein, Kalk, Kreide, Talkum, Bolus, Löß, Ton, Dolomit, Diatemeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte wie Getreidemehle, Baumrinden-, Holz- und Nußschalenmehl, Cellulosepulver und andere feste Trägerstoffe.

Zu den Mischungen oder Einzelwirkstoffen können Öle verschiedenen Typs, Herbizide, Fungizide, Nematozide, Insektizide, Bakterizide, Spurenelemente, Düngemittel, Antischaummittel (z.B. Silikone), Wachstumsregulatoren, Antidotmittel oder andere wirksame Verbindungen, eingesetzt werden.

**Patentansprüche für die Vertragsstaaten: BE CH DE FR GB IT LI LU NL SE**

1. Parabansäurederivate der Formel

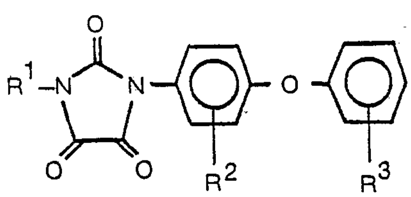

in der
$R^1$ Alkyl mit 1 bis 4 C-Atomen,
$R^2$ Halogen und
$R^3$ Alkyl mit 1 bis 3 C-Atomen, Halogen, Methoxy, Trifluormethoxy, Difluormethoxy oder Chlortrifluorethoxy bedeutet.

2. Parabansäurederivat gemäß Anspruch 1, ausgewählt aus der Gruppe bestehend aus 1 - (3′ - Chlor - 4′ - (3 - methoxyphenoxy) - phenyl) - 3 - methylparabansäure, 1 - (3′ - Chlor - 4′ - (4 - chlor - phenoxy) - phenyl) - 3 - methylparabansäure.

3. Herbizid, enthaltend ein Parabansäurederivat gemäß Anspruch 1.

4. Herbizid, enthaltend ein Parabansäurederivat gemäß Anspruch 2,.

5. Herbizid, enthaltend ein Parabansäurederivat gemäß Anspruch 1 sowie einen festen oder flüssigen Trägerstoff.

6. Verfahren zur Herstellung eines Herbizids, dadurch gekennzeichnet, daß man einen festen oder flüssigen Trägerstoff mit einem Parabansäurederivat gemäß Anspruch 1 vermischt.

7. Verfahren zur Bekämpfung unerwünschten Pflanzenwuchses, dadurch gekennzeichnet, daß, man die Pflanzen oder den Boden mit einem Parabansäurederivat gemäß Anspruch 1 behandelt.

**Patentansprüche für den Vertragsstaat: AT**

1. Herbizid, enthaltend ein Parabansäurederivat der Formel

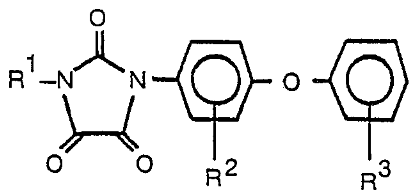

in der
$R^1$ Alkyl mit 1 bis 4 C-Atomen,
$R^2$ Halogen und
$R^3$ Alkyl mit 1 bis 3 C-Atomen, Halogen, Methoxy, Trifluormethoxy, Difluormethoxy oder Chlortrifluorethoxy bedeutet.

2. Herbizid gemäß Anspruch 1, enthaltend ein Parabansäurederivat ausgewählt aus der Gruppe bestehend aus 1-(3'-Chlor-4'-(3-methoxy-phenoxy)-phenyl)-3-methylparabansäure. 1-(3'-Chlor-4'-(4-chlor-phenoxy)-phenyl-3-methylparabansäure.

3. Herbizid, enthaltend einen festen oder flüssigen Trägerstoff sowie ein gemäß Anspruch 1 definiertes Parabansäurederivat.

4. Verfahren zur Herstellung eines Herbizids, dadurch gekennzeichnet, daß man einen festen oder flüssigen Trägerstoff mit einem gemäß Anspruch 1 definierten Parabansäurederivat vermischt.

5. Verfahren zur Bekämpfung unerwünschten Pflanzenwuchses, dadurch gekennzeichnet, daß man die Pflanzen oder den Boden mit einem gemäß Anspruch 1 definierten Parabansäurederivat behandelt.

**Claims for the Contracting States: BE CH DE FR GB IT LI LU NL SE**

1. A parabanic acid derivative of the formula

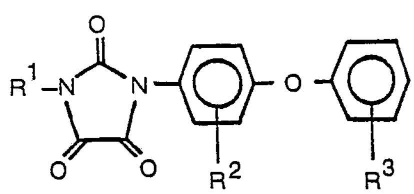

where $R^1$ denotes alkyl of 1 to 4 carbon atoms, $R^2$ denotes halogen and $R^3$ denotes alkyl of 1 to 3 carbon atoms, halogen, methoxy, trifluoromethoxy, difluoromethoxy or chlorotrifluoroethoxy.

2. A parabanic acid derivative as claimed in claim 1, selected from the group consisting of 1-(3'-chloro-4'-(3-methoxyphenoxy)-phenyl)-3-methylparabanic acid and 1-(3'-chloro-4'-(4-chlorophenoxy)-phenyl)-3-methylparabanic acid.

3. A herbicide containing a parabanic acid derivative as claimed in claim 1.

4. A herbicide containing a parabanic acid derivative as claimed in claim 2.

5. A herbicide containing a parabanic acid derivative as claimed in claim 1 and a solid or liquid carrier.

6. A process for the preparation of a herbicide, wherein a solid or liquid carrier is mixed with a parabanic acid derivative as claimed in claim 1.

7. A process for controlling the growth of unwanted plants, wherein the plants or the soil are treated with a parabanic acid derivative as claimed in claim 1.

**Claims for the Contracting State: AT**

1. A parabanic acid derivative of the formula

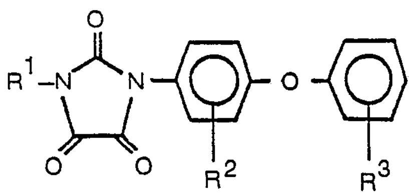

where $R^1$ denotes alkyl of 1 to 4 carbon atoms, $R^2$ denotes halogen and $R^3$ denotes alkyl of 1 to 3 carbon atoms, halogen, methoxy, trifluoromethoxy, difluoromethoxy or chlorotrifluoroethoxy.

2. A parabanic acid derivative as defined in claim 1, selected from the group consisting of 1-(3'-chloro-4'-(3-methoxyphenoxy)-phenyl)-3-methylparabanic acid and 1-(3'-chloro-4'-(4-chlorophenoxy)-phenyl)-3-methylparabanic acid.

3. A herbicide containing a solid or liquid carrier and a parabanic acid derivative as defined in claim 1.

4. A process for the preparation of a herbicide, wherein a solid or liquid carrier is mixed with a parabanic acid derivative as defined in claim 1.

5. A process for controlling the growth of unwanted plants, wherein the plants or the soil are treated with a parabanic acid derivative as defined in claim 1.

**Revendications pour les Etats contractants: BE CH DE FR GB IL LI LU NL SE**

1. Dérivés de l'acide parabanique de la formule

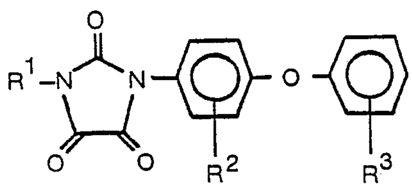

dans laquelle
$R^1$ désigne un radical alcoyle en $C_1$ à $C_4$,
$R^2$ un atome d'halogène et
$R^3$ un radical alcoyle en $C_1$ à $C_3$, un atome d'halogène ou un groupe méthoxy, trifluorométhoxy, difluorométhoxy ou chloro-trifluor-éthoxy.

2. Dérivé de l'acide parabanique suivant la revendication 1, choisi parmi l'acide 1 - (3' - chloro - 4' - (3 - méthoxy - phénoxy) - phényl) - 3 - méthyl - parabanique et l'acide 1 - (3' - chloro - 4' - (4 - chloro - phénoxy) - phényl) - 3 - méthyl - parabanique.

3. Composition herbicide, contenant un dérivé de l'acide parabanique selon la revendication 1.

4. Composition herbicide, contenant un dérivé de l'acide parabanique selon la revendication 2.

5. Composition herbicide, contenant un dérivé de l'acide parabanique selon la revendication 1, ainsi qu'une matière support solide ou liquide.

6. Procédé de préparation d'une composition herbicide, caractérisé en ce que l'on mélange un dérivé de l'acide parabanique selon la revendication 1 et une matière support solide ou liquide.

7. Procédé de lutte contre le développement de plantes indésirables, caractérisé en ce que l'on traite les plantes ou le sol avec un dérivé de l'acide parabanique selon la revendication 1.

**Revendications pour l'Etat contractant: AT**

1. Composition herbicide, contenant un dérivé de l'acide parabanique de la formule

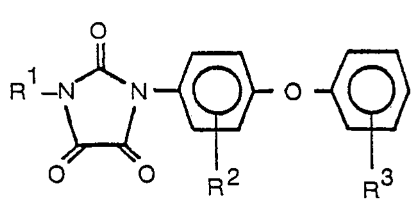

dans laquelle

$R^1$ désigne un radical alcoyle en $C_1$ à $C_4$,
$R^2$ un atome d'halogène et
$R^3$ un radical alcoyle en $C_1$ à $C_3$, un atome d'halogène ou un groupe méthoxy, trifluorométhoxy, difluorométhoxy ou chloro-trifluor-éthoxy.

2. Composition herbicide suivant la revendication 1, contenant un dérivé de l'acide parabanique choisi parmi l'acide 1 - (3' - chloro - 4' - (3 - méthoxy - phénoxy) - phényl) - 3 - méthyl - parabanique et l'acide 1 - (3' - chloro - 4' - (4 - chloro - phénoxy) - phényl) - 3 - méthyl - parabanique.

3. Composition herbicide, contenant un dérivé de l'acide parabanique tel que défini dans la revendication 1, ainsi qu'une matière support solide ou liquide.

4. Procédé de préparation d'une composition herbicide, caractérisé en ce que l'on mélange un dérivé de l'acide parabanique tel que défini dans la revendication 1 et une matière support solide ou liquide.

5. Procédé de lutte contre le développement de plantes indésirables, caractérisé en ce que l'on traite les plantes ou le sol avec un dérivé de l'acide parabanique tel que défini dans la revendication 1.